(19)

Europäisches
Patentamt
European
Patent Office
Office européen
des brevets

(11) **EP 4 059 550 A1**

(12) **EUROPEAN PATENT APPLICATION**
published in accordance with Art. 153(4) EPC

(43) Date of publication:
**21.09.2022 Bulletin 2022/38**

(21) Application number: **20888557.4**

(22) Date of filing: **11.11.2020**

(51) International Patent Classification (IPC):
**A61M 11/00** (2006.01)    **A61M 13/00** (2006.01)
**A61B 17/34** (2006.01)    **B05B 12/00** (2018.01)
**B05B 17/06** (2006.01)

(52) Cooperative Patent Classification (CPC):
**A61M 11/005; A61M 13/00; A61M 37/0092;**
A61B 17/3474; A61M 15/02; A61M 2205/3344;
A61M 2205/3368

(86) International application number:
**PCT/BR2020/000015**

(87) International publication number:
**WO 2021/092666 (20.05.2021 Gazette 2021/20)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **12.11.2019 BR 102019023835**

(71) Applicants:
• **Bhio Supply Indústria e Comércio de
Equipamentos Médicos Ltda.
93290-010 Porto Alegre - RS (BR)**
• **PIPAC BRASIL DESENVOLVIMENTO E
EXPLORAÇÃO DE INSTRUMENTO MEDICINAL
LTDA.
Porto Alegre (RS) (BR)**

(72) Inventors:
• **FERREIRA, Paulo Roberto Walter
93040-000 São Leopoldo (RS) (BR)**

• **DA SILVA JUNIOR, Edison Martins
92310-200 Canoas (RS) (BR)**
• **MIRANDA, Ivan
91110-410 Porto Alegre (RS) (BR)**
• **DOS SANTOS, Marcelo Saraiva
90240-100 Porto Alegre (RS) (BR)**
• **GRABOSKI, Moisés Dias
93224-290 Sapucaia do Sul (RS) (BR)**
• **DE MOURA LINCK, Viviane
90420-001 Porto Alegre (RS) (BR)**
• **SEITENFUS, Rafael
90630-090 Porto Alegre (RS) (BR)**
• **DE BARROS, Eduardo Dipp
91910-290 Porto Alegre (RS) (BR)**
• **POTT, Madison Ricardo
13561-002 São Carlos (SP) (BR)**
• **MORETTI, Thiago Balan
13575-450 São Carlos (SP) (BR)**

(74) Representative: **Pereira Garcia, João Luís
Simões, Garcia, Corte-Real e Associados
Rua Castilho, 167, 2°
1070-050 Lisboa (PT)**

(54) **ULTRASONIC AEROSOLIZATION PLATFORM FOR THE APPLICATION OF THERAPEUTIC SUBSTANCES TO BODY CAVITIES**

(57)    The present invention relates to an ultrasonic aerosolization platform that has a single access port (10) provided with a trocar (11) inserted into the surgical cavity through a single incision and provided with at least one internal channel (101) in which is positioned an ultrasonic aerosolizer (20) provided with a head (21) that houses a power piezoelectric transducer (214) and a resonating rod (22) that extends orthogonally from the head (21), provided with an internal channel (221) in communication with the internal channel (212) of the head (21) and a free end (222) with an atomization nozzle (30) with orifices (31); a high-frequency ultrasound generator (40) that provides an electrical signal to the power piezoelectric transducer (214), which converts said electrical signal into mechanical oscillations transmitted to the resonating rod (22) in the form of mechanical standing waves; and a processing unit (50) provided with an interface (60) for adjusting the excitation frequency of the transducer (214), adjusting the flow of the therapeutic substance, and adjusting the operating and actuation time of the electrode (not shown) arranged in the resonating rod (22).

EP 4 059 550 A1

Figure 1

**Description**

**FIELD OF THE INVENTION**

The present patent application describes an ultrasonic aerosolization platform for the application of therapeutic substances to body cavities. More specifically it comprises a single access port which allows the introduction of the endoscope instrument and administration of the aerosolized therapeutic substance by ultrasound in an intracavitary and/or intraperitoneal space and/or organs, by means of continuous and/or pulsed infusion, with possibility of modulation of the particle size during the procedure and modification of the physical properties of the therapeutic substance, such as electric charging and heating.

**BACKGROUND OF THE INVENTION**

Peritoneal carcinomatosis is considered as being an advanced neoplastic disease. The treatments reserved for these patients barely change the fatal outcome. In the last 20 years, the therapeutic approach of this condition has undergone important changes. The best understanding of the condition as part of a neoplastic dissemination process and a disease that is limited to just one "organ" - the peritoneum - changed the treatment scenery of this pathology. This new concept, developed from the studies by Dr. Paul H. Sugarbaker, led to different directions in the approach with patients with peritoneal carcinomatosis in neoplasia of the gastrointestinal tract, gynecologic and primary of the peritoneum. The association of the surgical cytoreduction and application of intraperitoneal chemotherapy become the key point in the attempt to control the pathology. The use of intraperitoneal chemotherapy appears in this scenario as one of the pillars of this new therapy. The direct application of the chemotherapeutic in the intraperitoneal space has shown results that are superior to the systemic chemotherapy when evaluating characteristics such as: drug concentration in the peritoneal space, penetration in the peritoneal metastasis and in the toxicity of the chemotherapy. The direct contact of the chemotherapeutic in the peritoneal space with the metastatic nodules has a superior bioactivity over the tumors when compared to the action of the systemic chemotherapy, demonstrating an advantage in the intraperitoneal application in the treatment of carcinomatosis.

In the intraperitoneal chemotherapy, the peritoneal space is bathed by liquid solutions carrying the chemotherapeutic agent. However, this manner of application presents limitations in the homogeneous distribution in the cavity and in the capacity of tissue penetration.

As an alternative to the common method of application of the intra-abdominal chemotherapy, the state of the art describes a new application embodiment named PIPAC (*Pressurized Intraperitoneal aerosol chemotherapy*}, where the chemotherapeutic solution is aerosolized, in order to enhance the distribution effects and the depth of penetration of the chemotherapeutic agent in the tissue due to the fact that the aerosol assumes the physical, behavioral, and distribution characteristics of a gas and the generation of pressure of the pneumoperitoneum [Nadiradze G, Giger-Pabst U, Zieren J, Strumberg D, Solass W, Reymond MA. Pressurized Intraperitoneal Aerosol Chemotherapy (PIPAC) with low-dose Cisplatin and Doxorubicin in gastric peritoneal metastasis. J Gastrointest Surg. 2016;20(2):367-73]. The intraperitoneal pressure modifies the so-called peritoneal permeability, changing the hydrostatic forces in the tissue, doubling the concentration of intraperitoneal substances in the extracellular space and increasing by five times the hydraulic conductivity of the fluid, leading it to the peritoneal metastases nucleus [Zakaria el-R, Lofthouse J, Flessner MF. In vivo hydraulic conductivity of muscle: effects of hydrostatic pressure. Am J Physiol. 1997;273(6 Pt 2):H2774~82.] and [Zakaria ER, Lofthouse J, Flessner MF. In vivo effects of hydrostatic pressure on interstitium of abdominal wall muscle. Am J Physiol. 1999;276(2 Pt 2):H517-29.]. This consolidates the concept of therapeutic pneumoperitoneum as a mechanism that is superior to all the other types of intraperitoneal chemotherapy release up to this moment [Seitenfus, Rafael, et al. Pressurised intraperitoneal aerosol chemotherapy (PIPAC) by single-port: alternative application in the control of peritoneal metastases. Rev. Gol Bras. Cir. vol.45 no.4 Rio de Janeiro, Epub Aug 20, 2018.].

However, recently published data show that the spatial distribution of the medicines throughout the abdominal cavity, based on the current technology P1 PAC-MIP®, where a microinjection pump is used, is not as homogeneous as expected [Khosrawipour, V.; Khosrawipour, T.; Diaz-Carballo, D.; Förster, E.; Zieren, J.; Giger-Pabst, U. Ann. Surg. OncoL 2016, 23, 1220-1224,doi:10.1245/sl 0434-015-4954-9], [Khosrawipour, V.; Khosrawipour, T.; Kern, A. J. P.; Osma, A.; Kabakci, B.; Diaz-Carballo, D.; Förster,E.; Zieren, J.; Fakhrian, K. J. CancerRes. Clin. OncoL 2016, 142, 2275-2280. doi:10.1007/s00432-016-2234-0], [Khosrawipour, V.; Khosrawipour, T.; Falkenstein, T. A.; Diaz-Carballo, D.; Förster, E.: Osma, A.; Adamietz, L A.; Zieren, J.; Fakhrian, K. Anticancer Res. 2016, 36, 4595-4600. doi:10.21873/anticanres.11008], [Khosrawipour, V.; Diaz-Carballo, D.; Ali-Haydar, A.; Khosrawipour, T.; Falkenstein, T. A.; Wu, D.; Zieren, J.; Giger-Pabst, U. World J. Surg. OncoL 2017, 15, 43. doi:10.1186/s12957-017- 1109-4], [Göhler, D.; Khosrawipour, V.; Khosrawipour, T.; Diaz-Carballo, D.; Falkenstein, T. A.; Zieren, J,; Stintz, M.; Giger-Pabst, U. Surg. Endosc.2QV7, 31, 1778-1784. doi:10.1007/s00464-016-5174-5] and [Bellendorf, A.; Khosrawipour, V.; Khosrawipour, T.: Siebigteroth, S.; Cohnen, J.; Diaz-Carballo, D.; Bockisch, A.; Zieren, J.; Giger-Pabst, U. Surg. Endosc. 2017.

doi:10.1007/s00464-017-5652-4]. The in-depth penetration in the analyzed tissue as well as the scintigraphic peritoneography showed access points on the side opposite to the outlet nozzle of the MIP® and insufficiently supplied regions, such as lateral areas to the aerosol jet. The lack of homogeneity in the drug deposition is attributed to the drop particle size and the aerosol drop kinetics generated by the MIP® [Göhler, D.; Khosrawipour, V.; Khosrawipour, T.; Diaz-Carballo, D.; Falkenstein, T. A.; Zieren, J.; Stintz, M.; Giger-Pabst, U. Surg. Endosc. 2017, 31, 1778-1784. doi:10.1007/s00464-016-5174-5]. Granulometric analyses of the MIP® aerosol by laser diffraction spectrometry showed that around 97,5% by volume of the aerosol is comprised by droplets between 3-200 μm with a volume weighted modal drop size of -25 μm. Said droplets are deposited immediately on the opposite side of the nozzle outlet due to the gravitational sedimentation and inertial impaction. Thus, Göhler et al. [Göhler, D.; Khosrawipour, V.; Khosrawipour, T.; Diaz-Carballo, D.; Falkenstein, T. A.; Zieren, J.; Stintz, M.; Giger-Pabst, U. Surg. Endosc. 2017, 31, 1778-1784. doi:10.1007/s00464-016-5174-5] concluded that the fraction of thick droplets based on the current PIPAC-MIP technology is too large to provide a homogeneous distribution of medicines.

The administration of aerosolized fluids in body cavities can be carried out by means of the single-port multifunctional platform described in document BR102018075741, wherein it is foreseen a single access port for the endoscopic instrument and the application of aerosolized and pressurized liquid solutions in any intracavitary or intraperitoneal space, as well as the execution of biopsy and visualization of the abdominal cavity, by means of the association with a shutter and an aerosolization device which generates droplets having a diameter of less than 150μm.

KAKCHEKEEVA [Kakchekeeva, Tinatin, et al. In Vivo Feasibility of Electrostatic Precipitation as an Adjunct to Pressurized Intraperitoneal Aerosol Chemotherapy (ePIPAC). Ann Surg Oncol, 2016. DO110.1245/s10434-016-5108-4] compares the PIPAC technique with the e-PIPAC, which comprises the electrostatic charging of the aerosolized particles. In this technique, the aerosolized therapeutic solution that is injected in the intraperitoneal space is electrostatically charged by an electrode that is positioned in the abdominal cavity of the patient which emits an electron current, resulting in the creation of negative gaseous ions. The gas ions collide with the particulate material, transmitting the negative charge. A return electrode grants a weak positive charge in the abdominal cavity, which results in the electrostatic attraction of the negatively charged aerosol particles on the surfaces of the peritoneum tissue. According to tests carried out in pigs, the ePIPAC improved the capture of two marker substances of the peritoneal tissue, having potential to allow a more efficient absorption of the drug and reduction of the application time.

More recently, there is described the HINAT (*Hyperthermic Intracavitary Nanoaerosol Therapy*), technology, detailed in document DEI 02016202316, which is based on the extra-cavitary generation of a hyperthermic and unipolar charge comprised by droplets of nanometric size provided to the abdominal cavity by means of an intracavitary access door, such as a trocar or Veress needle.

As described by Göhler [Göhler, Daniel, et al. Hyperthermic intracavitary nanoaerosol therapy (HINAT) as an improved approach for pressurised intraperitoneal aerosol chemotherapy (PIPAC): Technical description, experimental validation and first proof of concept. Beilstein J. Nanotechnol. 2017, 8, 2729-2740. doi:10.3762/bjnano.8.272], the heating of the aerosol to a temperature between 41 and 43 ºC causes the increase in the flow rate due to the reduction of dynamic viscosity, density and surface tension of the liquid, being reported increases in the penetration of the medicine due to the reduction in the intra-tumoral pressure and increase of the medicines deposition due to the improvement in the thermophoretic conditions. The analysis of the scintigraphic peritoneography (SPG) and analysis of the depth of multilocal penetration in the tissue (ITP) executed in *post-mortem* pigs disclosed a nearly uniform deposition of particles in the entire peritoneum post-death and a deeper penetration of the drug with less local variation when compared with the PIPAC-MIP approach.

Document DE102017006185 describes a device for the oriented introduction of a substance in a cavity of the body, said device having a first trocar for inflating a gas in the cavity and at least one second trocar connected to a substance container and distal extremity provided with a nozzle for the atomization of the substance. The respective proximal extremities of the at least two trocars, are fluidly connected to one another by means of a respective gas line, forming a closed circulation and providing a circulation circuit for a mixture of gas and inflating substance.

More recently, the state of the art describes transducers that can be applied in a liquid atomizer in order to generate a fine mist, such as disclosed in documents US4153201 and US3861852, wherein a pair of ultrasonic amplifiers having a quarter wavelength and a driving element sandwiched between the amplifiers is provided, and a second amplifying section having half wavelength which extends from one end of the first section and has theoretical resonant frequency equal to the actual resonant frequency of the first section. When used as a liquid atomizer the small diameter portion of the half-length amplifying section, which has a flanged nozzle, provides an atomizing surface of increased area. These ultrasonic atomizing nozzles are connected to an ultrasonic generator, such as described in document US9242263, which includes an amplifier for outputting a drive signal to the ultrasonic atomizing nozzle and a microcontroller, coupled to the amplifier, to control an output power of the amplifier.

Considering the countless embodiments for intraperitoneal chemotherapy release (hyperthermy, electric charging of therapeutic substance, aerosolization, among others) that are currently available to enhance the action of therapeutic agents, several studies persist in the sense of improving the therapeutic indexes in the treatment of tumors and metastases, common in several forms of abdominal cancer. The intraperitoneal administration of medicines increases the exposure of the cancer cells to the drug and minimizes the toxic effect for other organs [Kakchekeeva, Tinatin, et al. In Vivo Feasibility of Electrostatic Precipitation as an Adjunct to Pressurized intraperitoneal Aerosol Chemotherapy (ePIPAC). Ann Surg Oncol, 2016. DOI 10.1245/s10434-016-5108-4].

However, there are still required adjustments to provide the application of intracavitary drugs in a more homogeneous form, as well as the deeper penetration of the drugs in the tumor tissue, caused primarily by the rapid decrease of the concentration of the therapeutic substance, below the necessary level for destroying the cancer cells, wherein a large part of the residual tumor burden is not treated or is under-treated due to the limited exposure. The injection aerosolization equipment (or mechanical) require injection flows under pressure (> 150 PCI) greater than 25 ml/min to trigger stable aerosolization within the range of 0.7-110 microns, with an average 25 microns, as demonstrated by Gohler et al. [GOHLER, Daniel, et al. Hyperthermic intracavitary nanoaerosol therapy (HINAT) as an improved approach for pressurised Intraperitoneal aerosol chemotherapy (PIPAC): Technical description, experimental validation and first proof of concept. Beilstein J. Nanotechnol. 2017, 8, 2729-2740.]. This injection velocity is reached after 5 seconds, which determines a loss of the initial aerosolization of the procedure.

Moreover, the ultrasound aerosolization has important advantages regarding the aerosolization by injection. The frequencies above 30 kHz do not require injection flows under pressure (> 150 PCI) greater than 25 ml/min to reach the aerosolization, which occurs by direct transfer of the energy to the liquid substance, in an immediate manner as from the activation of the ultrasound. Additionally, the range of the size of the aerosol particle produced by ultrasound is narrower than the one verified in the range of the aerosol obtained by the conventional aerosolization equipment by injection under pressure, where devices of 45 kHz produce a range that varies from 13 to 43 microns, with an average of 15 microns, reaching a narrower and constant range when compared to the aerosolization devices by injection under pressure. However, in an innovative manner, the ultrasonic aerosolization devices allow for modulating the particle size during the application by means of power adjustment, which condition does not exist in the aerosolization apparatus by injection and pressure.

In this manner, it is an object of the present invention a platform comprising a single access port for the endoscopic instrument access and the administration of an aerosolized therapeutic substance to any intracavitary and/or intraperitoneal space and/or organs, with the possibility of obtaining particles having constant and/or variable size and capable of being modified in the physical properties thereof, such as temperature and electric charging, maintaining the therapeutic mist constant for a longer period of time, in order to increase the exposure time of the therapeutic substance to a neoplastic cell, as well as the action range, in order to reach the entirety of the cavity.

## SUMMARY

The invention provides an ultrasonic aerosolization platform for the application of therapeutical substances to body cavities which contains a single access port to the body cavity with an ultrasonic aerosolizer device for administering a therapeutic substance interlinked to an electronic module that allows for modulating both the size of the therapeutic mist droplet as the temperature of the substance and the electrophysical characteristic of the particles, without the need for pressure injection equipment or other equipment for altering the characteristics of the therapeutic particle.

The invention provides an ultrasonic aerosolization platform for the application of therapeutic substances to body cavities that allows for modulating the particle size and the distribution frequency, according to the therapeutic substance and the treatment, forming a stable and long-lasting therapeutic mist and preventing the condensation of the particles.

The invention provides an ultrasonic aerosolization platform for the application of therapeutic substances to body cavities that allows for modulating the particle size in the form of pulses in one sole application as well as the consistency of the particle size.

The invention provides an ultrasonic aerosolization platform for the application of therapeutic substances to body cavities that has a nozzle having a specific geometry for the mono and/or multidirectional dispersion of the aerosolized therapeutic substance and the cavitation throughout the body cavity.

The invention provides an ultrasonic aerosolization platform for the application of therapeutic substances to body cavities where it is possible to heat the therapeutic substance by means of the ultrasonic flow, reaching temperatures between 40 to 45 ºC, reducing the dynamic viscosity, density and surface tension of the liquid, with an increase in the penetration of the medication, as reported in the technical literature.

The invention provides an ultrasonic aerosolization platform for the application of therapeutic substances to body cavities that allows for executing the electric charging of the particle with effects proven in the technical literature [Reymond M, Demtroeder C, Solass W, Winnekendonk G, Tempfer G.Electrostatic precipitation Pressurized IntraPeritoneal Aerosol Chemotherapy (ePIPAC): first in-human application. Pleura Peritoneum. 2016 Jun 1;1 (2):109-116].

The invention provides an ultrasonic aerosolization platform for the application of therapeutic substances to body cavities that allows for controlling the delivery flow of the therapeutic substance in the space to be treated and the time of application, by means of adjustment in the micro processed module of the high-frequency ultrasound generator (40), functional characteristic not evidenced in the aerosolization devices of the state of the art.

## BRIEF DESCRIPTION OF THE FIGURES

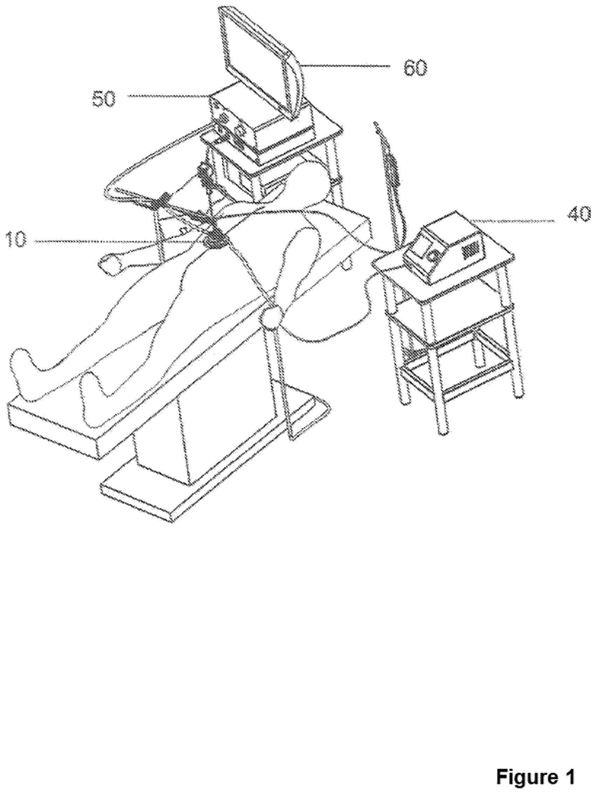

Figure 1 presents a representation of the ultrasonic aerosolization platform for the application of therapeutic substances to body cavities.

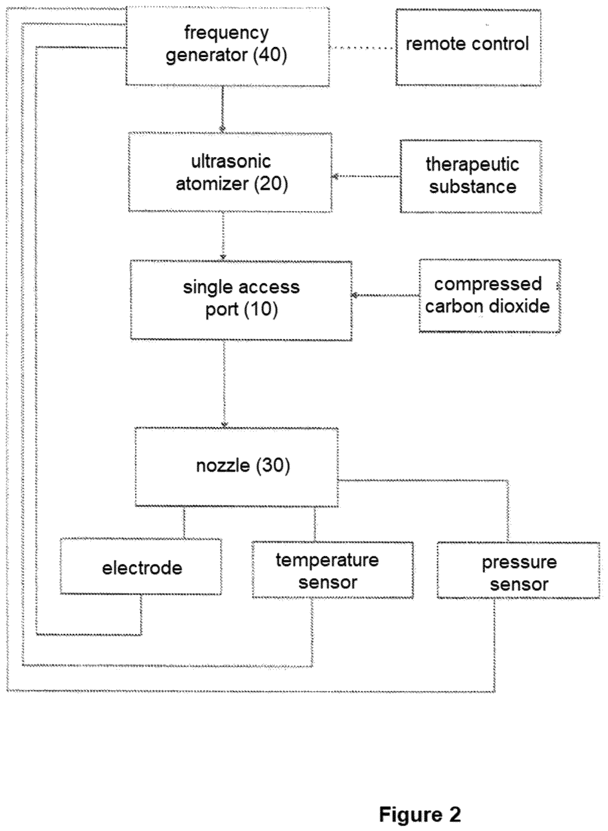

Figure 2 presents a schematic diagram of the ultrasonic aerosolization platform for the application of therapeutic substances to body cavities.

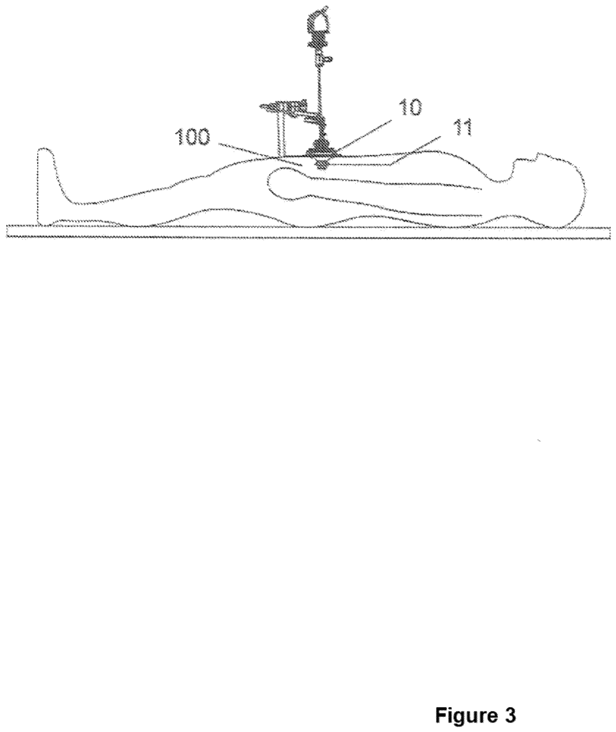

Figure 3 presents a cross-sectional view showing the single access port positioned in the body cavity.

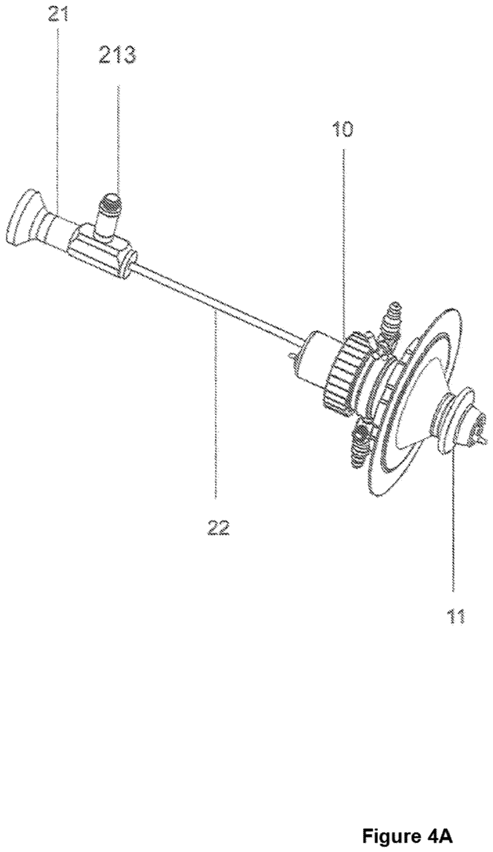

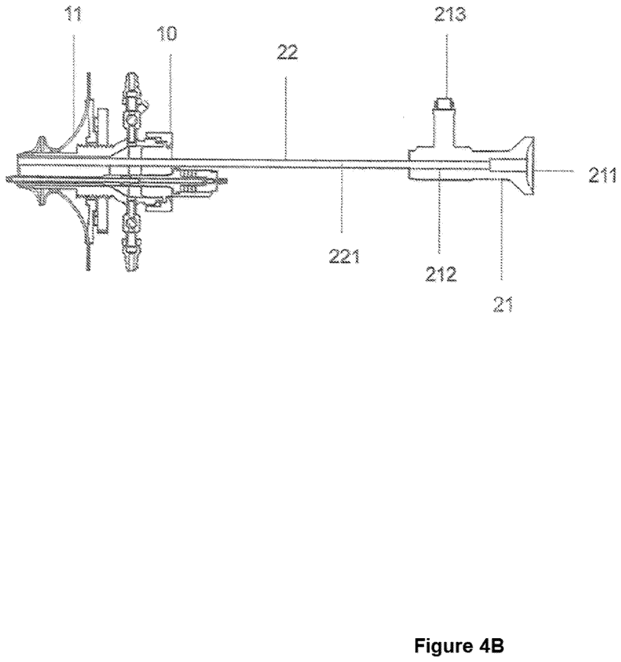

Figure 4A presents a perspective view showing the intracavitary single access port with the ultrasonic aerosolizer coupled and figure 4B presents a cross-sectional view.

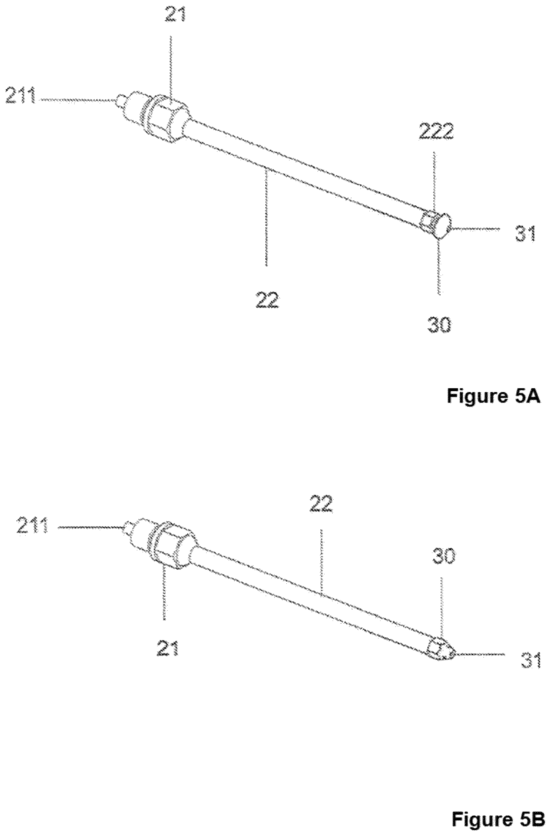

Figures 5A and 5B present details of the nozzle coupled to the ultrasonic aerosolizer.

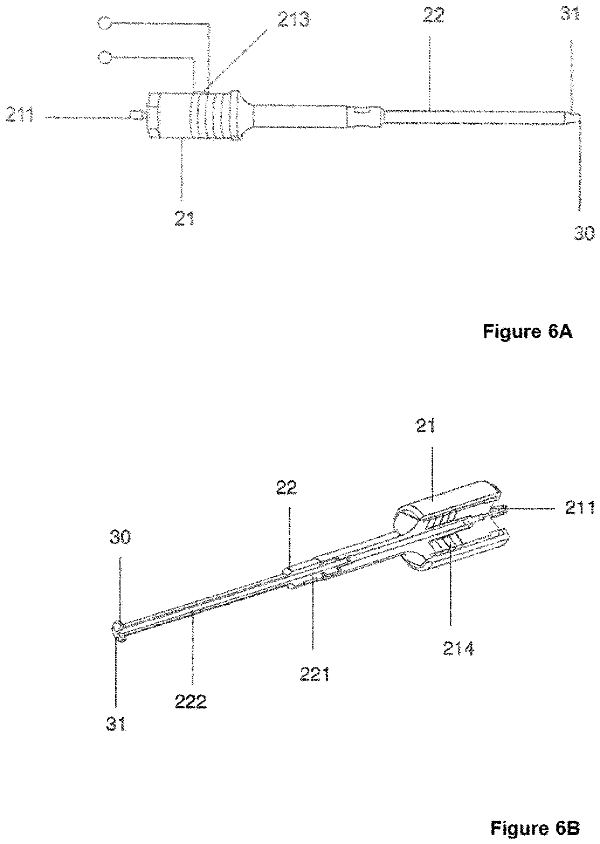

Figure 6A presents a representation of the ultrasonic aerosolizer and figure 6B presents details of the piezoelectric transducer arranged on the head of the aerosolizer.

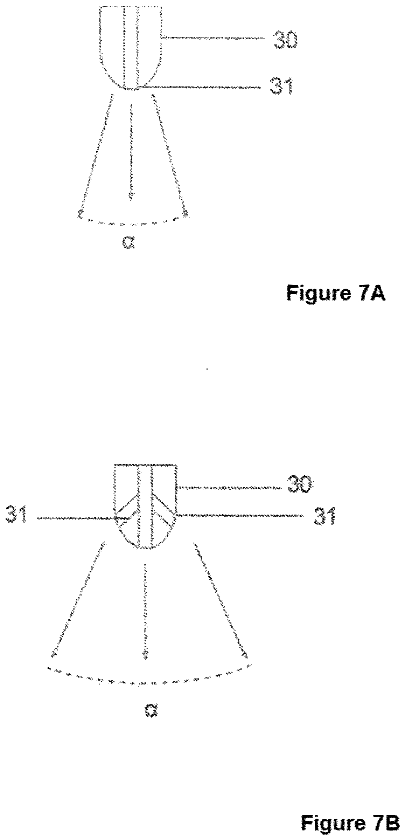

Figure 7A presents a representation of the unidirectional nozzle, and figure 7B presents a representation of the multidirectional nozzle, wherein $\alpha$ is the outlet angle of the therapeutic mist.

Figure 8 presents the graph of the relation of the particle size according to the frequency.

## DETAILED DESCRIPTION OF THE INVENTION

In the context of the present specification, the following expressions are conceptualized:

"therapeutic substance" comprises an active agent in liquid form, pharmaceutically acceptable for being administered to human beings selected among antibodies, chemotherapy, nanoparticulates, anti-adhesive, heat activated agents, among others.

"therapeutic mist" comprises an aerodispersoid constituted by liquid particles formed by mechanical rupture of a liquid and may also be denominated in the context of the present invention as being an aerosolized therapeutic substance.

The ultrasonic aerosolization platform for the application of therapeutic substances to body cavities, object of the present patent application, comprises a single access port (10) that allows the introduction of an endoscopic instrument and the administration of the aerosolized therapeutic substance in intracavitary and/or intraperitoneal space and/or organs of human beings by means of an ultrasonic aerosolizer (20) interlinked to an electronic module that allows for modulating both the size of the droplet of the therapeutic mist as the temperature of the substance and the electrophysical characteristic of the particles, without the need for equipment for pressure injection or other equipment for changing the characteristics of the therapeutic particle.

The ultrasonic aerosolization platform for the application of therapeutic substances in body cavities, compared to the HINAT Technologies *(Hyperthermic Intracavitary Nanoaerosol Therapy)* and PIPAC-MIP is presented in Table 1 as follows.

## Table 1: comparative of the parameters of the HINAT, PIPAC-MIP technologies and the platform object of the present invention.

| Parameter | HINAT | PIPA-MIP | Platform object of the invention |
|---|---|---|---|
| Number of components | 2 | 2 | 1 |
| Diameter of the aerosolizer (mm) | 0.5 | 0.2 | 01-0.2 |
| Particle size (µm) | 0.7-20 | 0.7-110 | 13-43 |
| Average particle size (µm) | 1 | 20 | 15 |
| Application speed of the liquid (l/h) | 210-500 | 1250-3000 | 1.8-450 |
| Pressure (Bar) | 1.0-3.0 | 6.10-13.8 | Not applicable |
| Particle modularization | no | no | yes |
| Variable flow | no | no | yes |
| Heating of liquid | no | no | yes |
| Control of time of application | no | no | yes (3-30 min.) |

This single access port (10), described in document BR102012021227 and used in the multifunctional single port platform disclosed in document BR102018075741, basically comprises a trocar (11) inserted in the surgical cavity by means of a single incision, said trocar (11) having at least one internal channel (101) which extends between the distal and proximal ends, wherein in said proximal end a quick connector is provided for the coupling of a seal having at least one orifice for the positioning of the instrumentals in the internal channel (101) so that the active ends remain positioned in the body cavity (100). In the proximity of the proximal end of the trocar (11) a valve is foreseen with a first route for inflation of carbon dioxide ($CO_2$) to form the pneumoperitoneum and, optionally, a second route for the carbon dioxide exhaust. Considering that the single access port has already been described in previous patents, the technical details related to said single access port (10) will be disregarded in this document, presenting solely the characteristics that are necessary for the sufficient description of the aerosolization platform invention, which is the object of the present invention.

In the internal channel (101) of the single access port (10) an ultrasonic aerosolizer (20) is positioned wherein the liquid therapeutic substance is decomposed into droplets, generating a therapeutic mist to be dissipated in body cavities (100) by means of a nozzle (30) positioned on the distal end of the internal channel (101) of the port (10) which is found in the body cavity.

The ultrasonic aerosolizer (20) basically comprises a structure having a head (21) from which a resonating rod (22) extends provided with an internal channel (221) which receives the liquid therapeutic substance by means of a valve (211) and, free end (222) wherein an atomization nozzle (30) is arranged provided with orifices (31) for forming the therapeutic mist in the body cavity (100).

The valve (211) has a quick connector or similar wherein a tube is coupled in order to allow the inlet of the liquid therapeutic substance.

In the head (21) connectors (213) are foreseen for coupling the high-frequency ultrasound generator (40) which provides energy to the aerosolizer (20).

The head (21) houses a power piezoelectric transducer (214) which electrical signal received from the high-frequency ultrasound generator (40) is converted into mechanical oscillations. As is known to a person skilled in the art, a piezoelectric transducer (214) is constituted by ceramics compressed by two metallic masses by means of a pre-tensioning screw which maintains said ceramics compressed. The ceramics are polarized longitudinally and the polarization directions are alternating for each ceramic in the assembly of the transducer and excited by two metallic electrodes, placed one on each one of the faces thereof. When an electrical signal is applied to this transducer (214) the field that is created causes corresponding deformations in the ceramic, making it vibrate strongly and thus generate sound waves in the corresponding frequency.

The high-frequency sound waves generated in the transducer (214) are transmitted to the resonating rod (22) in the form of mechanical standing waves, creating a type of nodes and anti-nodes, having a "whiplash" effect which increases the vibration amplitude. In this manner, the therapeutic substance in liquid form which enters through the valve (211) of the head (21) and crosses through the internal channel (212) of said head (21), when entering the internal channel (221) of the resonating rod (22) in the direction of the extreme portion (222) decomposes into uniform droplets of micrometric size, forming a therapeutic mist to be released by the orifices (31) of the nozzle (30) to be dissipated in the body cavity (100) by means of cavitation.

The nozzle (30) arranged in the free end (222) of the resonating rod (22) can be fixed or interchangeable, allowing to adapt nozzles (30) of several shapes that allows modifying the shape, the outlet volume and/or the aerosolized particle size to be dissipated by the orifices (31), to direct the therapeutic mist generated in the direction of the base and the walls of the body cavity (100), as described by Dobre & Bolle (Dobre, M. and Bolle L. Practical design of ultrasonic spray devices: experimental testing of several atomizer geometries. Experimental Thermal and Fluid Science. Elsevier, 2002).

The high-frequency ultrasound generator (40) that provides energy to the power piezoelectric transducer (214), is interlinked to a processing unit (50) provided with a computer program and a database with a set of instructions, said processing unit (50) which identifies the natural frequency of the transducer (214) and stimulates it in the previously adjusted working frequency, to obtain the maximum energetic efficiency, allowing the modulation of the size of the particle of the therapeutic mist by means of the adjustment of the excitation frequency of the transducer (214) as will be described next.

By means of an interface (60), the operator adjusts the parameters of the processing unit (50) to control, for example, the flow of the therapeutic liquid substance in the ultrasonic aerosolizer (20) by means of intervention at the speed of the peristaltic pump (not shown) installed in the feeding line of the therapeutic substance to the aerosolizer (20), providing continuous and/or pulsed infusion, as well as adjusting the delivery/dissipation time of the aerosolized substance in the body cavity (100).

For the electric charging to the particles of therapeutic mist, there is foreseen an electrode (not shown) on the resonating rod (22) which issues an electron current which collides with the aerosolized therapeutical substance promoting the electric charging of the particles. A positively charged blanket is provided on the body surface of the patient which electrostatically attracts the particles to the surface of the body cavity in treatment, guaranteeing the adhesion and the depth, said electrode being fed by means of the supply of energy from the high-frequency ultrasound generator (40).

For the distance actuation of the high-frequency ultrasound generator (40), a remote control is foreseen.

The mechanical waves that propagate on the resonating rod (22) generate heat (caused by the interaction between the incident wave and the reflected wave) which can be used to provide the heating of the therapeutic substance which dislocates in the internal channel (221) of the resonating rod (22). Thus, the flow of the therapeutic substance can be adjusted on the peristaltic pump (not shown) of the ultrasound generator (40) flows in the order of 1.8 l/h to 450 l/h being obtained, obtaining a temperature between 25 ºC to 50 ºC. Additionally, the temperature is intrinsically connected to the size of the particle [Avvaru, B. et all. Ultrasonic atomization: Effect of liquid phase properties. Ultrasonics 44 146-158. Elsevier, 2006].

The average particle size of the therapeutic mist is obtained by means of Equation 1, based on LANG (Lang, R. J., Ultrasonic atomization of liquids, J. Acoust. Soc. Am., Vol. 34, No. 1, 1962, pp. 6- 8).

$$\text{Equation 1: } D_g = 0.34 \times 10^6 \times \left( \frac{8 \times \pi \times \gamma}{\rho \times f^2} \right)^{1/3}$$

Where:

Dg = average diameter of the particle/drop ($\mu$)
Y = surface tension of the liquid (N/m)
$\rho$ = density of the liquid at 20 ºC (kg/m3)
f = excitation ultrasound frequency (Hz)

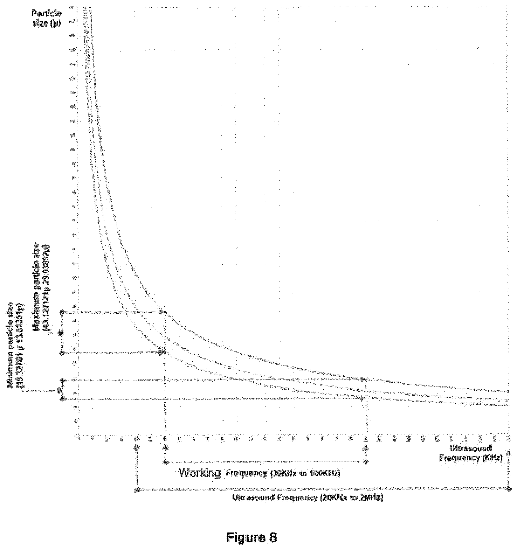

According to the increase of the excitation frequency of the transducer (214), the size of the particle diminishes, in other words, the average particle size of the aerosolized mist is inversely proportional to the frequency. In the working frequency from 30KHz to 100KHz, the particle diameter can be at least 13 microns and at most 43 microns, as evidenced in figure 8.

Optionally, on the resonating rod (22) or on the port (10) at least one temperature sensor is arranged (not shown) which sends signals to the processing unit (50) to control the temperature in the body cavity (100), indicating, by means of an alarm, reaching a distinct temperature from the one previously parametrized.

Optionally, in the port (10) at least one pressure sensor is arranged (not shown) which sends signals to the processing unit (50) to control the pressure in the body cavity (100).

Optionally, the ultrasonic aerosolizer (20) is positioned in video-surgery sheaths having from 10 to 12 mm diameter, the use of the single access port (10) being dispensed with.

The processing unit (50) can be coupled to the ultrasound generator (40), providing a single structure.

**Claims**

1. ULTRASONIC AEROSOLIZATION PLATFORM FOR THE APPLICATION OF THERAPEUTIC SUBSTANCES TO BODY CAVITIES that has a single access port (10) provided with a trocar (11) inserted into the surgical cavity by means of a single incision and provided with at least one internal channel (101) that extends between the proximal and distal ends, wherein in said proximal end a quick connector is arranged for the coupling of a seal having at least one orifice for positioning the instruments in the internal channel (101), a valve having a first route for inflation of carbon dioxide (CO2) in the proximal end of the trocar (11) and, optionally, a second route for the carbon dioxide exhaust, **characterized in that** it has:

   a) an ultrasonic aerosolizer (20) positioned on the internal channel (101) of the single access port (10) provided with a head (21) that houses a power piezoelectric transducer (214), a valve (211) which connects with the internal channel (212) arranged on the head (21) and connectors (213) for coupling the power cable of the high-frequency ultrasound generator (40); and a resonating rod (22) which projects orthogonally from the head (21) provided with an internal channel (221) in communication with the internal channel (212) of the head (21) and free end (222) having an atomizing nozzle (30) with orifices (31);
   b) a high-frequency ultrasound generator (40) that provides an electrical signal to the power piezoelectric transducer (214) that converts said electrical signal into mechanical oscillations transmitted to the resonating rod (22) in the form of mechanical standing waves;
   c) a processing unit (50) provided with a computer program and a database with a set of instructions, said processing unit (50) provided with an interface (60) for adjusting the excitation frequency of the transducer (214), adjusting the flow of the therapeutic substance by means of the activation of the peristaltic pump (not shown) installed in the feeding line of the therapeutic substance to the aerosolizer (20), adjusting the operation and actuation time of the electrode (not shown) arranged in the resonating rod (22).

2. ULTRASONIC AEROSOLIZATION PLATFORM FOR THE APPLICATION OF THERAPEUTIC SUBSTANCES TO BODY CAVITIES, according to claim 1, **characterized in that** the nozzle (30) is fixed or interchangeable at the free end (222) of the resonating rod (22).

3. ULTRASONIC AEROSOLIZATION PLATFORM FOR THE APPLICATION OF THERAPEUTIC SUBSTANCES TO BODY CAVITIES, according to claim 1, **characterized in that** the electrode (not shown) issues an electron current that collides with the aerosolized therapeutic substance in the internal channel (221) of the resonating rod (22).

4. ULTRASONIC AEROSOLIZATION PLATFORM FOR THE APPLICATION OF THERAPEUTIC SUBSTANCES TO BODY CAVITIES, according to claim 1, **characterized in that** it has a temperature sensor (not shown) installed on the resonating rod (22) or on the access port (10) which sends data to the processing unit (50).

5. ULTRASONIC AEROSOLIZATION PLATFORM FOR THE APPLICATION OF THERAPEUTIC SUBSTANCES TO BODY CAVITIES , according to claim 1, **characterized in that** it has a pressure sensor (not shown) arranged in the trocar (11) of the access port (10) which sends data to the processing unit (50).

Figure 1

**Figure 2**

100    10    11

**Figure 3**

Figure 4B

**Figure 5A**

**Figure 5B**

211

213    22    31

21

**Figure 6A**

21

22

30

211

214

221

222

31

**Figure 6B**

Figure 7A

Figure 7B

Figure 8

## INTERNATIONAL SEARCH REPORT

| International application No. |
| --- |
| **PCT/BR2020/000015** |

A.    CLASSIFICATION OF SUBJECT MATTER

   **IPC: A61M 11/00 (2006.01),  A61M 13/00 (2006.01),  A61B 17/34 (2006.01),  B05B 12/00 (2018.01),  B05B 17/06 (2006.01)**

According to International Patent Classification (IPC) or to both national classification and IPC

B.    FIELDS SEARCHED

Minimum documentation searched (classification system followed by classification symbols)

   **A61M 11/00, A61M 13/00, A61B 17/34, B05B**

Documentation searched other than minimum documentation to the extent that such documents are included in the fields searched

**INPI-BR patent database**

Electronic data base consulted during the international search (name of data base and, where practicable, search terms used)

**EPODOC**

C.    DOCUMENTS CONSIDERED TO BE RELEVANT

| Category* | Citation of document, with indication, where appropriate, of the relevant passages | Relevant to claim No. |
| --- | --- | --- |
| A | WO 2011018777 A1 ( HYLAND KIERAN [IE]) <br> 17 **February** 2011 (2011-02-17) <br> (   **See entire document** ) <br> ------------------------------------------------ | 1 – 5 |
| A | US 2012234321 A1 ( CLANCY DERMOT [IE]) <br> 20  **September**  2012 (2012-09-20) <br> (  **See entire document**   ) <br> ------------------------------------------------ | 1 – 5 |
| A | WO 2004043274 A1 ( TARAN DAVID [IL]) <br> 27 **May**  2004 (2004-05-27) <br> (   **See entire document**  ) <br> ------------------------------------------------ | 1 – 5 |
| A | US 4153201 A (SONO TEK CORP) <br> 08 **May**  1979 (1979-05-08) <br> (  **See entire document**   ) <br> ------------------------------------------------ | 1 – 5 |

|   | Further documents are listed in the continuation of Box C. | [X] | See patent family annex. |
| --- | --- | --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **28/01/2021** | 09/02/2021 |

| Name and mailing address of the ISA/ <br> INSTITUTO NACIONAL DA PROPRIEDADE INDUSTRIAL <br> Rua Mayrink Veiga nº 9, 6º andar <br> cep: 20090-910, Centro - Rio de Janeiro/RJ <br> Facsimile No.   +55 21 3037-3663 | Authorized officer <br> **Walter Edgley de Oliveira** <br><br> Telephone No.   +55 21 3037-3493/3742 |
| --- | --- |

Form PCT/ISA/210 (second sheet) (January 2015)

**INTERNATIONAL SEARCH REPORT**
Information on patent family members

International application No.

**PCT/BR2020/000015**

| | | | |
|---|---|---|---|
| WO 2011018777 A1 | 2011-02-17 | EP 2464403 A1 | 2012-06-20 |
| | | US 2012209166 A1 | 2012-08-16 |
| | | US 8551036 B2 | 2013-10-08 |
| | | US 2015057600 A1 | 2015-02-26 |
| | | US 9629967 B2 | 2017-04-25 |
| | | US 2017224938 A1 | 2017-08-10 |
| | | US 10166348 B2 | 2019-01-01 |
| | | US 2019160236 A1 | 2019-05-30 |
| | | US 10888674 B2 | 2021-01-12 |
| | | US 2011030678 A1 | 2011-02-10 |
| | | US 2014121589 A1 | 2014-05-01 |
| US 2012234321 A1 | 2012-09-20 | EP 2139409 A1 | 2010-01-06 |
| | | US 2008236577 A1 | 2008-10-02 |
| | | US 2008243050 A1 | 2008-10-02 |
| | | US 2009235925 A1 | 2009-09-24 |
| | | US 2009240192 A1 | 2009-09-24 |
| | | US 2009241948 A1 | 2009-10-01 |
| | | US 2011178458 A1 | 2011-07-21 |
| | | US 2012192863 A1 | 2012-08-02 |
| | | WO 2008117264 A1 | 2008-10-02 |
| | | WO 2008117265 A1 | 2008-10-02 |
| WO 2004043274 A1 | 2004-05-27 | AU 2003279504 A1 | 2004-06-03 |
| | | IL 152813 D0 | 2003-06-24 |
| US 4153201 A | 1979-05-08 | AT A797277 A | 1986-12-15 |
| | | AT 383509 B | 1987-07-10 |
| | | BE 860540 A | 1978-05-08 |
| | | CA 1071997 A | 1980-02-19 |
| | | CA 1090694 A | 1980-12-02 |
| | | CA 1090695 A | 1980-12-02 |
| | | CH 627097 A5 | 1981-12-31 |
| | | DE 2749859 A1 | 1979-05-10 |
| | | DK 475677 A | 1978-05-09 |
| | | DK 150229 B | 1987-01-12 |
| | | ES 463976 A1 | 1980-12-16 |
| | | FI 773325 A | 1978-05-09 |
| | | FR 2386226 A1 | 1978-10-27 |
| | | GB 1595715 A | 1981-08-19 |
| | | GB 1595716 A | 1981-08-19 |
| | | GB 1595717 A | 1981-08-19 |
| | | IE 46066 L | 1979-05-08 |
| | | IE 822450 L | 1978-05-08 |
| | | IE 46067 B1 | 1983-02-09 |
| | | IE 822451 L | 1978-05-08 |
| | | IE 46068 B1 | 1983-02-09 |
| | | IL 53328 D0 | 1978-04-30 |
| | | IT 1090915 B | 1985-06-26 |
| | | JP S5359929 A | 1978-05-30 |
| | | JP S5816082 B2 | 1983-03-29 |
| | | JP S5892480 A | 1983-06-01 |
| | | LU 78476 A1 | 1978-03-14 |

Form PCT/ISA/210 (patent family annex) (January 2015)

INTERNATIONAL SEARCH REPORT
Information on patent family members

International application No.

PCT/BR2020/000015

|  |  | MX 148756 A | 1983-06-14 |
|  |  | NL 7712249 A | 1978-05-10 |
|  |  | NL 186796 B | 1990-10-01 |
|  |  | NO 773808 L | 1978-05-09 |
|  |  | NO 148826 B | 1983-09-12 |
|  |  | PT 67246 A | 1977-12-01 |
|  |  | SE 7712563 L | 1978-05-09 |
|  |  | SE 434348 B | 1984-07-23 |
|  |  | US 4301968 A | 1981-11-24 |
|  |  | ZA 776376 B | 1978-10-25 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- BR 102018075741
- DE 102017006185
- US 4153201 A
- US 3861852 A
- US 9242263 B
- BR 102012021227

### Non-patent literature cited in the description

- **NADIRADZE G ; GIGER-PABST U ; ZIEREN J ; STRUMBERG D ; SOLASS W ; REYMOND MA.** Pressurized Intraperitoneal Aerosol Chemotherapy (PIPAC) with low-dose Cisplatin and Doxorubicin in gastric peritoneal metastasis. *J Gastrointest Surg,* 2016, vol. 20 (2), 367-73
- In vivo hydraulic conductivity of muscle: effects of hydrostatic pressure. **ZAKARIA EL-R ; LOFTHOUSE J ; FLESSNER MF.** Am J Physiol. 6, 1997, vol. 273, H2774-82
- **ZAKARIA ER ; LOFTHOUSE J ; FLESSNER MF.** In vivo effects of hydrostatic pressure on interstitium of abdominal wall muscle. *Am J Physiol.,* 1999, vol. 276 (2), H517-29
- **RAFAEL et al.** Pressurised intraperitoneal aerosol chemotherapy (PIPAC) by single-port: alternative application in the control of peritoneal metastases. *Rev. Gol Bras. Cir.,* 20 August 2018, vol. 45 (4
- **KHOSRAWIPOUR, V. ; KHOSRAWIPOUR, T. ; DIAZ-CARBALLO, D. ; FÖRSTER, E. ; ZIEREN, J. ; GIGER-PABST, U.** *Ann. Surg. OncoL,* 2016, vol. 23, 1220-1224
- **KHOSRAWIPOUR, V. ; KHOSRAWIPOUR, T. ; KERN, A. J. P. ; OSMA, A. ; KABAKCI, B. ; DIAZ-CARBALLO, D. ; FÖRSTER,E. ; ZIEREN, J. ; FAKHRIAN, K.** *J. CancerRes. Clin. OncoL,* 2016, vol. 142, 2275-2280
- **KHOSRAWIPOUR, V. ; KHOSRAWIPOUR, T. ; FALKENSTEIN, T. A. ; DIAZ-CARBALLO, D. ; FÖRSTER, E. ; OSMA, A. ; ADAMIETZ, L A. ; ZIEREN, J. ; FAKHRIAN, K.** *Anticancer Res.,* 2016, vol. 36, 4595-4600
- **KHOSRAWIPOUR, V. ; DIAZ-CARBALLO, D. ; ALI-HAYDAR, A. ; KHOSRAWIPOUR, T. ; FALKENSTEIN, T. A. ; WU, D. ; ZIEREN, J. ; GIGER-PABST, U.** *World J. Surg. OncoL,* 2017, vol. 15, 43
- **GÖHLER, D. ; KHOSRAWIPOUR, V. ; KHOSRAWIPOUR, T. ; DIAZ-CARBALLO, D. ; FALKENSTEIN, T. A. ; ZIEREN, J ; STINTZ, M. ; GIGER-PABST, U.** *Surg. Endosc.2QV7,* vol. 31, 1778-1784
- **BELLENDORF, A. ; KHOSRAWIPOUR, V. ; KHOSRAWIPOUR, T. ; SIEBIGTEROTH, S. ; COHNEN, J. ; DIAZ-CARBALLO, D. ; BOCKISCH, A. ; ZIEREN, J. ; GIGER-PABST, U.** *Surg. Endosc.,* 2017
- **GÖHLER, D. ; KHOSRAWIPOUR, V. ; KHOSRAWIPOUR, T. ; DIAZ-CARBALLO, D. ; FALKENSTEIN, T. A. ; ZIEREN, J. ; STINTZ, M. ; GIGER-PABST, U.** *Surg. Endosc.,* 2017, vol. 31, 1778-1784
- **GÖHLER, D. ; KHOSRAWIPOUR, V. ; KHOSRAWIPOUR, T. ; DIAZ-CARBALLO, D. ; FALKENSTEIN, T. A. ; ZIEREN, J. ; STINTZ, M. ; GIGER-PABST, U.** *Surg. Endosc.,* 2017, vol. 31, 1778-1784
- **KAKCHEKEEVA, TINATIN et al.** In Vivo Feasibility of Electrostatic Precipitation as an Adjunct to Pressurized Intraperitoneal Aerosol Chemotherapy (ePIPAC). *Ann Surg Oncol,* 2016
- **GÖHLER, DANIEL et al.** Hyperthermic intracavitary nanoaerosol therapy (HINAT) as an improved approach for pressurised intraperitoneal aerosol chemotherapy (PIPAC): Technical description, experimental validation and first proof of concept. *Beilstein J. Nanotechnol.,* 2017, vol. 8, 2729-2740
- **KAKCHEKEEVA, TINATIN et al.** In Vivo Feasibility of Electrostatic Precipitation as an Adjunct to Pressurized intraperitoneal Aerosol Chemotherapy (ePIPAC. *Ann Surg Oncol,* 2016
- **GOHLER, DANIEL et al.** Hyperthermic intracavitary nanoaerosol therapy (HINAT) as an improved approach for pressurised Intraperitoneal aerosol chemotherapy (PIPAC): Technical description, experimental validation and first proof of concept. *Beilstein J. Nanotechnol.,* 2017, vol. 8, 2729-2740
- **REYMOND M ; DEMTROEDER C ; SOLASS W ; WINNEKENDONK G ; TEMPFER G.** Electrostatic precipitation Pressurized IntraPeritoneal Aerosol Chemotherapy (ePIPAC): first in-human application. *Pleura Peritoneum,* 01 June 2016, vol. 1 (2), 109-116

- Practical design of ultrasonic spray devices: experimental testing of several atomizer geometries. **DOBRE, M. ; BOLLE L.** Experimental Thermal and Fluid Science. Elsevier, 2002

- Ultrasonic atomization: Effect of liquid phase properties. **AVVARU, B.** Ultrasonics. Elsevier, 2006, vol. 44, 146-158
- **LANG, R. J.** Ultrasonic atomization of liquids. *J. Acoust. Soc. Am.,* 1962, vol. 34 (1), 6-8